(19) Europäisches Patentamt
European Patent Office
Office européen des brevets

(11) Numéro de publication : **0 076 756**
**B1**

(12) # FASCICULE DE BREVET EUROPÉEN

(45) Date de publication du fascicule du brevet : **04.03.87**

(21) Numéro de dépôt : **82401794.1**

(22) Date de dépôt : **01.10.82**

(51) Int. Cl.[4] : **C 07 D231/20**, C 07 D403/12, A 61 K 31/415

(54) **Dérivés du pyrazole, procédé pour leur préparation et composition pharmaceutiques les contenant.**

(30) Priorité : **02.10.81 FR 8118596**

(43) Date de publication de la demande : **13.04.83 Bulletin 83/15**

(45) Mention de la délivrance du brevet : **04.03.87 Bulletin 87/10**

(84) Etats contractants désignés : **BE CH DE FR GB IT LI LU NL**

(56) Documents cités :
**EP-A- 0 007 019**
**EP-A- 0 037 344**

(73) Titulaire : **ETABLISSEMENTS NATIVELLE S.A.**
**27, rue de la Procession**
**F-75015 Paris (FR)**

(72) Inventeur : **Jarreau, François-Xavier**
**5 rue Louis Hervé**
**F-78000 Versailles (FR)**
Inventeur : **Koenig, Jean-Jacques**
**31, rue du Panorama**
**F-77670 Vernou-la-Celle s/seine (FR)**

(74) Mandataire : **L'Helgouaich, Jean et al**
**OFFICE PICARD 134 Boulevard de Clichy**
**F-75018 Paris (FR)**

**EP 0 076 756 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

La présente invention, réalisée dans les laboratoires du CERES — Centre Européen de Recherche Scientifique — concerne de nouveaux dérivés du pyrazole, qui peuvent être appliqués en thérapeutique, et un procédé pour leur préparation.

La demande de brevet EP 0 007 019 décrit des dérivés du 5-phénylpyrazole substitués en position 3 par un groupe du type alcoxycarbonyl-alkyl-2-oxy ou aminocarbonyl-alkyl-2-oxy, utilisables en thérapeutique comme hypolipidémiants.

Les dérivés du pyrazole conformes à l'invention sont des aryl-3 alcoxy-5 pyrazoles représentés par la formule générale (I) :

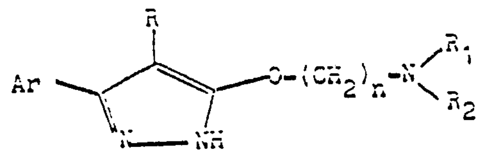

(I)

dans laquelle Ar représente un groupe phényle, ou un groupe phényle substitué par un ou plusieurs atomes d'halogène ou groupes alkyle, alcoxy, cyano, nitro, ou hydroxy ; R représente un atome d'hydrogène ou un groupe alkyle, phényle ou benzyle ; $R_1$ et $R_2$, identiques ou différents, représentent un atome d'hydrogène ou un groupe alkyle inférieur de 1 à 4 atomes de carbone, ou forment avec l'atome d'azote auquel ils sont rattachés, un hétérocycle de 5 à 7 chaînons comportant le cas échéant un ou plusieurs autres hétéroatomes choisis parmi l'azote et l'oxygène ; n est un entier de 1 à 4.

L'invention a également pour objet un procédé permettant de préparer les dérivés de formule générale (I) à partir de produits connus aisément accessibles, avec un bon rendement.

Les dérivés du pyrazole de formule générale (I) et leurs sels pharmaceutiquement acceptables peuvent être appliqués en thérapeutique humaine ou vétérinaire, notamment pour le traitement des arythmies cardiaques.

Le groupe phényle représenté par Ar dans la formule générale (I) peut être substitué par un ou plusieurs atomes d'halogène ou groupes alkyle, alcoxy, cyano, nitro ou hydroxy. R peut représenter un atome d'hydrogène ou un groupe alkyle linéaire ou ramifié, tel que méthyle, éthyle, isopropyle, n-butyle, t-butyle, isoamyle, etc., ou un groupe phényle ou benzyle.

Quand $R_1$ et $R_2$ forment un hétérocycle avec l'atome d'azote auquel ils sont rattachés, cet hétérocycle peut être un groupe complété par des atomes de carbone, et par exemple un groupe pyrrolyle, pyrrolidinyle, pipéridyle, azépinyle, etc. ; cet hétérocycle peut également contenir un ou plusieurs autres hétéroatomes choisis parmi l'azote et l'oxygène, et on peut citer par exemple les groupes oxazolidinyle, pyrazolyle, pipérazinyle, imidazolyle, morpholinyle, pyrazolinyle, etc.

Dans la formule générale (I) ci-dessus, Ar représente de préférence un groupe phényle, ou un groupe phényle substitué par un ou plusieurs atomes d'halogène tels que le chlore, le brome ou le fluor, et par exemple un groupe p-chlorophényle ou un groupe 3,4-dichlorophényle. R représente de préférence un atome d'hydrogène, un groupe alkyle inférieur de 1 à 4 atomes de carbone, tel qu'un groupe méthyle, éthyle ou propyle, ou un groupe benzyle. $R_1$ et $R_2$ représentent de préférence un groupe alkyle inférieur de 1 à 4 atomes de carbone, tel qu'un groupe méthyle ou isopropyle, $R_1$ pouvant être un atome d'hydrogène tandis que $R_2$ est un groupe alkyle, et inversement, ou forment ensemble un groupe hétérocyclique et plus particulièrement un groupe pyrrolidinyle, pipéridyle, oxazolidinyle, pipérazinyle, ou morpholinyle. n prend de préférence les valeurs 2 ou 3.

Les aryl-3 alcoxy-5 pyrazoles suivant la présente invention existent sous plusieurs formes représentées par la formule (I) ci-dessus et les formules (IA) et (IB) ci-dessous :

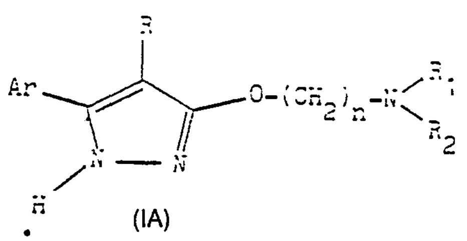

(IA)

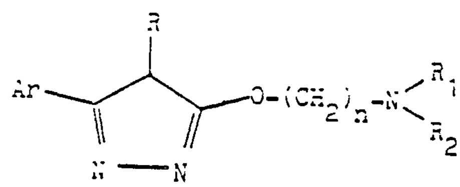

(IB)

Les formes (I) et (IA) sont prédominantes, mais des facteurs extérieurs peuvent déplacer les équilibres vers la forme (IB). L'invention concerne bien entendu les aryl-3 alcoxy-5 pyrazoles sous toutes les formes (I), (IA) et (IB) représentées ci-dessus.

L'invention concerne également les sels des dérivés du pyrazole, représentés par la formule générale (I) ci-dessus, et plus particulièrement les sels pharmaceutiquement acceptables, obtenus en faisant agir sur le dérivé un acide minéral ou organique usuel tel que l'acide chlorhydrique, sulfurique, lactique,

oxalique, citrique, phosphorique, stéarique, maléique, tartrique, etc. La réaction est réalisée suivant les techniques usuelles, l'acide et le dérivé réagissant généralement en proportions sensiblement stœchiométriques.

Les dérivés du pyrazole conformes à l'invention, représentés par la formule générale (I) peuvent être préparés à partir des pyrazolones de formule (II) :

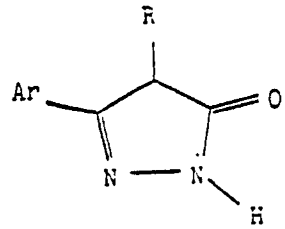

(II)

dans laquelle Ar et R ont la même signification que dans la formule (I), par réaction d'alkylation en milieu basique par une halogénoalkylamine de formule générale (III) :

$$X-(CH_2)_n-NR_1R_2 \quad \text{(III)}$$

dans laquelle X représente un atome d'halogène tel que le chlore ou le brome, et n, $R_1$ et $R_2$ conservent les définitions indiquées dans la formule générale (I).

Les aryl-3 pyrazolones de formule (II) utilisées comme produits de départ sont les composés connus qui peuvent être préparés suivant les techniques usuelles, comme décrit par exemple dans « The Chemistry of Heterocyclic Compounds » Weissberger Ed. (1964), Interscienсes Publ.

La réaction d'alkylation des aryl-3 pyrazolones de formule (II) par les halogénoalkylamines de formule (III) s'effectue en milieu basique en présence d'une base telle qu'un hydrure, un amidure, un carbonate ou un alcoolate de métal alcalin ou une amine, dans un solvant organique approprié.

La réaction peut s'effectuer par exemple en présence d'hydrure de sodium, d'amidure de sodium, d'hydrure de potassium, d'éthylate de sodium, de carbonate de potassium, de carbonate de sodium, de diéthylamine, de triéthylamine, etc. dans un solvant choisi parmi le diméthylformamide, le diméthylsulfoxyde, le dioxanne, le tétrahydrofuranne, un alcool tel que le méthanol ou l'éthanol, l'acétone. Lorsque le solvant est un alcool, il peut être avantageux d'utiliser comme base un alcoolate correspondant, et par exemple l'éthylate de sodium dans l'éthanol. Conformément à l'invention on utilise de préférence l'hydrure de sodium dans le diméthylformamide, le carbonate de potassium dans le dioxanne ou l'éthylate de sodium dans l'éthanol.

La réaction peut s'effectuer à température ambiante, mais il peut être préférable de chauffer le mélange réactionnel à une température comprise entre 30 et 100 °C, et de préférence entre 40 et 70 °C, pour accélérer la réaction.

Suivant une forme préférentielle de mise en œuvre du procédé de l'invention, on dissout la pyrazolone de formule (II) dans un solvant organique sous atmosphère d'azote, on ajoute une base telle que l'hydrure de sodium, le carbonate de potassium ou l'amidure de sodium, puis on chauffe légèrement et on ajoute progressivement l'halogénoalkylamine de formule (III). Le solvant est éliminé par évaporation sous pression réduite et on purifie le produit obtenu, par les techniques usuelles d'extractions acidobasiques ou de chromatographie.

Les halogénoalkylamines de formule (III) utilisées comme réactifs pour effectuer l'alkylation des pyrazolones de formule (II) conformément au procédé de l'invention, sont généralement disponibles dans le commerce sous forme de leurs chlorhydrates. Il peut être avantageux de transformer ces chlorhydrates en bases correspondantes, au moment de l'utilisation, en les dissolvant dans une solution saturée de carbonate de potassium et en effectuant une extraction, suivant la technique décrite dans Fieser & Fieser, « Reagents for Organic Synthesis » n° IV, John Wiley & Sons (1974).

Comme exemples d'halogénoalkylamines utilisables conformément à l'invention, on peut citer plus particulièrement la N-(chloro-2 éthyl)-diméthylamine, la N-(chloro-3 propyl)-diméthylamine, ou la N-(chloro-2 éthyl)-diisopropylamine, ou encore des halogénoalkylamines hétérocycliques telles que la N-(chloro-2 éthyl)-pyrrolidine, la N-(chloro-2 éthyl)-pipéridine, la N-(chloro-3 propyl)-morpholine, la N-(chloro-3 propyl)-pipéridine, etc.

Suivant une variante conforme à la présente invention, les dérivés du pyrazole de formule générale (I) peuvent être préparés à partir d'un aroylacétate d'éthyle de formule générale (IV) :

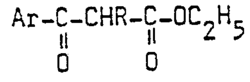

(IV)

où Ar et R ont la même signification que dans la formule (I), que l'on transforme par transestérification au moyen d'un aminoalcool de formule (V) :

$$HO-(CH_2)_n-NR_1R_2 \qquad (V)$$

où n, $R_1$ et $R_2$ ont la même signification que dans la formule (I), pour obtenir le composé de formule (VI) :

$$Ar-\underset{\underset{O}{\|}}{C}-CHR-\underset{\underset{O}{\|}}{C}-O-(CH_2)_n-NR_1R_2 \qquad (VI)$$

sur lequel on effectue une cyclisation au moyen d'hydrazine en milieu acide, procurant le dérivé de formule générale (I).

La transestérification permettant de transformer l'aroylacétate d'éthyle de formule (IV) en ester cétonique de formule (VI) s'effectue de préférence par chauffage dans un solvant permettant une distillation azéotropique ; de plus, il est avantageux d'opérer en présence d'un catalyseur acide. Le solvant peut être par exemple un hydrocarbure tel que le toluène ou le xylène.

La réaction de cyclisation par l'hydrazine en milieu acide, par exemple en milieu acide chlorhydrique concentré ou acide sulfurique, peut s'effectuer suivant une technique analogue à celle de H. J. Backer et W. Meier « Rec. Trav. Chim. P.B. » *45*, 428 (1926).

Les exemples suivants illustrent l'invention sans en limiter la portée.

## Exemple 1

p-chlorophényl-3 diméthylaminopropoxy-5 pyrazole.

Dans un ballon de 500 ml muni d'un agitateur, d'un thermomètre, d'un réfrigérant, d'une ampoule de coulée et d'une arrivée d'azote, on place 9,5 g de p-chlorophényl-3 pyrazolone dans 70 ml de dioxanne. On porte à reflux, sous atmosphère d'azote, et on ajoute 4 g de carbonate de potassium. Après 1 h de chauffage sous reflux, on ajoute 7,2 g de diméthylamino-1 chloro-3 propane en maintenant le milieu réactionnel sous reflux ; l'addition est effectuée progressivement en 45 mn. On maintient à reflux pendant environ 4 h puis on effectue des extractions acido-basiques suivant les techniques usuelles après élimination du solvant par évaporation sous vide.

Après recristallisation dans l'hexane, puis dans l'éther isopropylique, on obtient 6,6 g de p-chlorophényl-3 diméthylaminopropoxy-5 pyrazole (rendement 47 %)

Point de fusion : F = 114 °C (éther isopropylique)

Spectre IR (Nujol) $\nu$ = 3 130, 3 100, 1 510, 1 490 $cm^{-1}$

Spectre de RMN ($CDCl_3$) $\delta$ = 1,7-2,7 (4H), 2,2 (s, 6H), 4,0 (t, 2H), 5,8 (s, 1H), 7,3 (q, 4H), 11,2 (s, 1H) ppm

Par action de l'acide maléique dans l'éthanol, suivant les techniques usuelles, on prépare le maléate du composé ci-dessus.

Point de fusion : F = 155 °C

## Exemple 2

phényl-3 méthyl-4 pyrrolidinoéthoxy-5 pyrazole.

Comme dans l'exemple 1, on place 8 g de phényl-3 pyrazolone-5 dans 60 ml de dioxanne et on chauffe à reflux sous atmosphère d'azote, puis on ajoute 3,9 g de carbonate de potassium. On maintient le mélange réactionnel à reflux pendant environ 1 h, et on ajoute progressivement 13 g de N-(chloro-2 éthyl) pyrrolidine.

Après 4 h de réaction en maintenant le mélange à reflux, on élimine le solvant par évaporation sous vide, et on effectue une extraction.

Après recristallisation dans le cyclohexane, on obtient 3,5 g de phényl-3 méthyl-4 pyrrolidino-éthoxy-5 pyrazole (rendement 26 %).

Point de fusion : F = 88 °C (cyclohexane)

Chromatographie sur couche mince (CCM) :

Rf = 0,4 (acétate d'éthyle + 10 % diéthylamine)

Le maléate correspondant, obtenu par les techniques usuelles, présente un point de fusion F = 158-159 °C (éthanol).

## Exemple 3

p-chlorophényl-3 pipéridinopropoxy-5 pyrazole.

Dans un ballon de 500 ml muni d'un réfrigérant avec garde de chlorure de calcium, d'une ampoule de coulée, d'un thermomètre et d'une arrivée d'azote, on place 19,4 g de p-chlorophényl-3 pyrazolone dans

60 ml de diméthylformamide anhydre, puis on dégaze la solution en y faisant barboter un courant d'azote. On refroidit à environ 5 °C et on ajoute 2,8 g d'hydrure de sodium. On laisse la température du mélange remonter jusqu'à la température ambiante, puis on chauffe progressivement à 40 °C, on ajoute goutte à goutte 17,4 g de chloro-3 propylpipéridine et on laisse réagir pendant environ 10 h.

Après évaporation du solvant sous vide, extraction acido-basique, et recristallisation dans l'isopropanol, on obtient 10,7 g de p-chlorophényl-3 pipéridinopropoxy-5 pyrazole (rendement 30 %). Par ailleurs, on récupère, au cours de la purification, environ 25 % de la p-chlorophényl-3 pyrazolone-5 de départ ainsi que environ 3 g de p-chlorophényl-3 pipéridinopropyl-2 pyrazolone comme produit secondaire.

Point de fusion : F = 140 °C (isopropanol)

Spectre IR (Nujol) : $\nu$ = 3 250, 1 510 $cm^{-1}$

Spectre de RMN ($CDCl_3$) : $\delta$ = 1,6 (6H), 2,1 (2H), 2,6(6H), 4,3 (t, 2H), 6,2 (s, 1H), 7,8 (q, 4H) ppm.

### Exemple 4

phényl-3 méthyl-4 morpholinoéthoxy-5 pyrazole.

On procède comme dans l'exemple 1 en plaçant 8 g de phényl-3 pyrazolone-5 dans 60 ml de dioxanne. On chauffe à reflux, et on ajoute 4,1 g de carbonate de potassium, puis, progressivement, 7,8 g de N-(chloro-2 éthyl) morpholine.

Quand la réaction est terminée, après extraction et recristallisation dans l'isopropanol, on obtient 5,1 g de phényl-3 méthyl-4 morpholinoéthoxy-5 pyrazole (rendement 36 %)

Point de fusion : F = 100 °C (isopropanol)

CCM : Rf = 0,60 (acétate d'éthyle + 10 % diéthylamine)

Le maléate correspondant, préparé par la technique usuelle, par action de l'acide maléique dans l'éthanol, présente un point de fusion F = 156 °C.

### Exemple 5

phényl-3 morpholinoéthoxy-5 pyrazole.

Dans un ballon de 250 ml muni d'un appareil de distillation azéotropique et d'une ampoule de coulée, on dissout 9,6 g de benzoylacétate d'éthyle et 19,7 g de N-(hydroxy-2 éthyl) morpholine dans 100 ml de toluène.

On porte à ébullition, on distille l'azéotrope toluène-éthanol, et on ajoute en même temps du toluène pur. La réaction est pratiquement totale en 4 heures environ. On lave le toluène à l'eau distillée, puis à l'eau saturée de chlorure de calcium, et on sèche sur sulfate de sodium et on évapore à sec. On obtient ainsi 12,4 g de benzoylacétate de morpholinoéthyle (rendement 90 %) sous forme d'huile jaune.

Dans un ballon de 100 ml, on place 0,3 g d'hydrazine hydratée, 15 ml d'éthanol absolu et 1,6 ml d'acide chlorhydrique concentré. On chauffe à reflux et on ajoute 1,4 g de benzoylacétate de morpholinoéthyle obtenu comme indiqué ci-dessus. On maintient à reflux pendant environ 2 h, et la réaction est alors pratiquement totale. On évapore l'éthanol sous vide, puis on noie avec de l'eau et on extrait au chloroforme pour isoler 0,1 g environ d'éthoxy-5 phényl-3 pyrazole. La phase aqueuse est alcalinisée, puis extraite au chloroforme pour procurer 0,5 g de phényl-3 morpholinoéthoxy-5 pyrazole (rendement 36 %)

Point de fusion : F = 100 °C (isopropanol)

Spectre IR (Nujol) $\nu$ = 3 260, 1 595, 1 510 $cm^{-1}$

### Exemples 6 à 23

En procédant comme indiqué dans l'exemple 1, mais en modifiant de manière appropriée la pyrazolone et l'halogénoalkylamine de départ, on obtient les produits indiqués au tableau ci-après, ainsi que leurs sels, dont les caractéristiques (point de fusion, solvant de recristallisation et chromatographie sur couche mince avec un éluant constitué d'acétate d'éthyle additionné de 10 % de diéthylamine) sont également mentionnées au même tableau.

### Exemples 6 à 23

(Voir Exemples 6 à 23 pages 6 et 7)

Les expérimentations effectuées sur les aryl-3 alcoxy-5 pyrazoles conformes à l'invention ont permis de faire apparaître d'intéressantes propriétés pharmacologiques et toxicologiques justifiant leur application possible en thérapeutique vétérinaire et humaine.

Propriétés toxicologiques

Exemples 6 à 23

| Ex. | Ar | R | $NR_1R_2$ | n | F°C | Rf* | Sel | F°C |
|---|---|---|---|---|---|---|---|---|
| 6 | 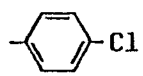 | H | 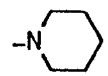 | 2 | 117 | 0,65 | tartrate | 212 |
| 7 | 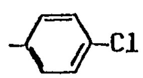 | H | 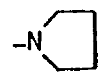 | 2 | 128 | 0,60 | maléate | 190 |
| 8 | 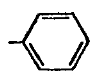 | H | $-N(CH_3)_2$ | 3 | (huile) | 0,45 | maléate | 115 |
| 9 | 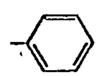 | H | 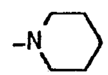 | 2 | 70-72 | 0,55 | maléate | 139 |
| 10 | 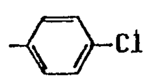 | H | 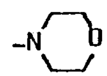 | 2 | 120 | 0,60 | maléate | 170 |
| 11 | 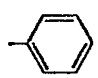 | $CH_3$ | 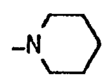 | 2 | 80 | 0,3 | maléate | 143 |
| 12 | 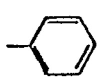 | H | 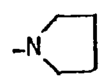 | 2 | (huile) | 0,35 | maléate | 131 |
| 13 | 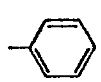 | H | 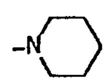 | 3 | (huile) | 0,55 | oxalate | 137 |
| 14 | 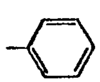 | $CH_3$ | 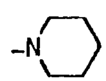 | 3 | (huile) | 0,5 | maléate | 116-117 |

(Suite)

| Ex. | Ar | R | $NR_1R_2$ | n | F°C | Rf* | Sel | F°C |
|---|---|---|---|---|---|---|---|---|
| 15 | 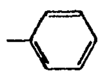 | $CH_3$ | $-N(CH_3)_2$ | 3 | (huile) | 0,3 | maléate | 155-158 |
| 16 | 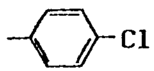 | $CH_3$ | 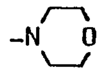 | 2 | 112 | 0,5 | chlorhydrate | 210 |
| 17 | 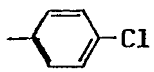 | $CH_3$ | $-N(CH_3)_2$ | 3 | 98 | 0,45 | tartrate | 71 |
| 18 | 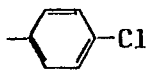 | $CH_3$ | 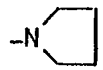 | 2 | 108 | 0,70 | maléate | 165 |
| 19 | 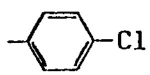 | $CH_3$ | 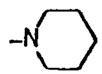 | 3 | 89 | 0,6 | tartrate | 102-118 |
| 20 | 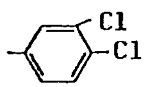 | H | 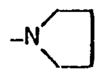 | 2 | 128 | 0,50 | maléate | 176 |
| 21 | 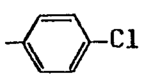 | $CH_3$ | 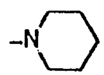 | 2 | 113 | 0,55 | tartrate | 186 |
| 22 | 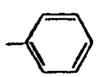 | $-CH_2-C_6H_5$ | 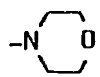 | 2 | (huile) | 0,45 | tartrate | 152-153 |
| 23 | 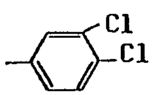 | H | 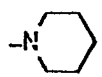 | 3 | 96 | 0,44 | maléate | 140 |

*C.C.M. : Acétate d'éthyle + 10 % diéthylamine

La toxicité aiguë des dérivés de l'invention a été étudiée par administration orale (P.O.) à la souris (10 animaux, 5 mâles et 5 femelles par dose) et calcul de la dose léthale 50 (DL 50) selon la méthode de Litchfield et Wilcoxon (J. Pharmacol. 1949, 96, 99-113). Le tableau 1 présente les valeurs de DL 50 des dérivés suivant l'invention.

Dans certains cas, la DL 50 a également été étudiée après injection intrapéritonéale (I.P.) des dérivés.

Tableau 1

| EXEMPLE n° | DL 50 P.O. mg/kg | DL 50 I.P. mg/kg |
|---|---|---|
| 1 | 1 000 | 250 à 500 |
| 2 | 500 | |
| 3 | 700 | |
| 4 | 750 | |
| 5 | 1 000 | |
| 6 | 500 | |
| 7 | 1 000 | 280 |
| 8 | 1 000 | |
| 9 | 400 à 800 | |
| 10 | 1 000 | |
| 11 | 250 à 500 | 184 |
| 12 | 500 à 1 000 | |
| 13 | 125 à 250 | |
| 14 | 250 | |
| 15 | 500 à 1 000 | |
| 16 | 500 à 1 000 | 125 à 250 |
| 17 | 500 à 1 000 | 250 à 500 |
| 18 | 750 | |
| 19 | 250 | 62 à 125 |

Propriétés pharmacologiques

1. Tolérance cardiovasculaire

La tolérance cardiovasculaire a été étudiée sur l'oreillette isolée de cobaye, sur le rat anesthésié et sur le chien anesthésié.

Sur l'oreillette isolée de cobaye, la préparation permet la mesure de la force contractile du myocarde auriculaire de cobaye et la fréquence des contractions auriculaires. A la concentration de 10 µg/ml de la solution du bain, les dérivés testés provoquent une diminution de la fréquence des contractions de 15 à 75 % (moyenne — 43,25 %) par rapport à la période contrôle pré-traitement. A la même concentration, la force de contraction varie de 0 à — 80 % par rapport à la période contrôle (variation moyenne — 37,5 %).

Sur le rat anesthésié, les dérivés suivant l'invention, administrés à des doses de 6 à 25 mg/kg, entraînent une diminution de la fréquence cardiaque (5 à 40 %), du débit aortique, et de la pression artérielle systolique (4 à 60 %).

Sur le chien anesthésié, les résultats obtenus en ce qui concerne la tolérance hémodynamique sont regroupés au tableau 2 ci-après qui indique les pourcentages de variation des paramètres hémodynamiques par rapport à la période contrôle pré-traitement pour les dérivés des exemples 2 et 4.

Les paramètres ont été enregistrés à l'aide de :

— Cathéters reliés à des capteurs de pression (pression artérielle, pression ventriculaire gauche et sa dérivée première dp/dt) ;

— débitmètre électromagnétique sur l'aorte (débit aortique) ;

— électrocardiographe ;

— jauge de contrainte placée sur la myocarde ventriculaire gauche (force de contraction myocardique).

Les dérivés sont injectés par voie intraveineuse, une dose toutes les 30 minutes environ et leurs effets sont mesurés 20 minutes après la fin de l'injection (durée de l'injection 2 minutes).

Tableau 2

Effets hémodynamiques chez le chien anesthésié

(les effets sont exprimés en pourcentage de variation par rapport à la période contrôle pré-traitement)

Exemple 4

| | DOSES (mg/kg) | | | | |
|---|---|---|---|---|---|
| | 1 | +3 | +10 | +20 | +40 |
| Pression artérielle systolique | 0 | 0 | 0 | 0 | 0 |
| Fréquence cardiaque | 0 | 0 | 0 | 0 | - 18 |
| Débit cardiaque | 0 | 0 | - 23 | - 34 | - 11 |
| Volume systolique | 0 | 0 | - 23 | - 34 | - 8 |
| Force contraction du myocarde | + 5 | + 10 | + 26 | + 42 | + 52 |
| (dp/dt)1/p | + 10 | + 2 | - 10 | - 21 | - 10 |

Exemple 2

| | DOSES (mg/kg) | | | | | |
|---|---|---|---|---|---|---|
| | 0,5 | + 1 | + 3 | + 5 | + 10 | + 10 |
| Pression artérielle systolique | + 4 | 0 | 0 | - 8 | - 12 | - 20 |
| Fréquence cardiaque | 0 | - 8 | - 8 | - 8 | - 8 | - 8 |
| Débit cardiaque | 0 | - 5 | - 5 | - 20 | - 37 | - 45 |
| Volume systolique | 0 | + 3 | + 3 | - 12 | - 31 | - 40 |
| Force de contraction du myocarde | 0 | 0 | - 20 | - 20 | - 10 | - 20 |
| (dp/dt)1/p | 0 | - 7 | - 30 | - 30 | - 38 | - 46 |

Les résultats exposés au tableau 2, ainsi que les résultats analogues obtenus avec les autres dérivés conformes à la présente invention montrent que les dérivés entraînent chez le chien les modifications suivantes :

— la pression artérielle systolique ne change pas (dérivés des exemples 4 et 9), ou baisse modérément (dérivés des exemples 2, 14 et 19). Ainsi, pour le dérivé n° 2, cette baisse n'apparaît qu'après la dose cumulée de 4,5 mg/kg ;

— la fréquence cardiaque ne change pas (dérivés des exemples 4 et 19), ou baisse modérément (dérivés des exemples 9, 2 et 14) ;

— le débit cardiaque s'abaisse dans tous les cas. Cependant cette modification reste modérée puisqu'une chute de 20 % n'est atteinte qu'avec des doses cumulées supérieures à 4 mg/kg (dérivés des exemples 4, 9 et 19) ou même supérieures à 9 mg/kg (dérivé de l'exemple 2) ;

— la force des contractions myocardiques ventriculaires augmente après les injections du dérivé n° 4, ne varie pas de façon significative avec les dérivés n° 9 et 14, diminue avec les dérivés n° 2 et 19. Là également cette diminution, lorsqu'elle existe, reste modérée (maximum de — 20 % pour le dérivé n° 2 après l'injection de 4,5 mg/kg à 29 mg/kg).

Ces résultats montrent que la tolérance cardiovasculaire chez le chien est satisfaisante puisque les effets sont limités à une chute modérée du débit cardiaque tandis que force des contractions myocardiques, pression artérielle systolique et fréquence cardiaque varient diversement.

2. Tests électrophysiologiques.

Le test de la maximum fréquence suivie étudie la fréquence maximum de stimulation qui peut entraîner la contraction d'une oreillette isolée de cobaye placée dans une solution contenant un dérivé conforme à l'invention. La mesure est effectuée avant (période contrôle), puis après introduction du dérivé dans la solution.

Le tableau 3 ci-après montre les pourcentages de diminution de la maximum fréquence suivie, pour une concentration de 10 mg/l de chacun des dérivés suivant l'invention. Les résultats obtenus montrent que les dérivés conformes à l'invention augmentent la période réfractaire auriculaire de 12 à 56 %.

Tableau 3

| Exemples | Δ % MFS |
|---|---|
| 1 | — 30 |
| 2 | — 56 |
| 3 | — 53 |
| 4 | — 27 |
| 5 | — 12 |
| 6 | — 46 |
| 7 | — 45 |
| 8 | — |
| 9 | — 48 |
| 10 | — 15 |
| 11 | — 52 |
| 12 | — 35 |
| 13 | — 44 |
| 14 | — 40 |
| 15 | — 40 |
| 16 | — 28 |
| 17 | — 32 |
| 18 | — 45 |
| 19 | — 52 |

De plus, l'étude électrophysiologique a été réalisée chez le chien anesthésié au pentobarbital, à thorax fermé, à l'aide de cathéters-électrodes bipolaires introduites dans les cavités cardiaques par voies veineuse et artérielle transcutanée, afin de mesurer l'automaticité sinusale, les temps de conduction intracardiaque, et les périodes réfractaires cardiaques effectives et fonctionnelles.

Lors de l'étude électrophysiologique, les dérivés sont injectés par voie intraveineuse en 2 minutes pour chaque dose et à 30 minutes d'intervalle. La mesure des différents paramètres est effectuée avant l'injection de la première dose (période contrôle) et de 10 à 28 minutes après l'injection de chaque dose de la substance. On constate que l'automaticité sinusale n'est pas significativement déprimée de même que la vitesse de conduction entre les oreillettes et le nœud auriculo-ventriculaire. Par contre, la conduction se ralentit dans le système His-Purkinje dès la dose de 4,5 mg/kg et on sait que ce paramètre est très caractéristique des effets des médicaments antiarythmiques du groupe I de Vaughan-Williams (quinidine-like). Les périodes réfractaires effectives auriculaires et ventriculaires s'allongent proportionnellement à la dose administrée. La période réfractaire fonctionnelle nodale auriculo-ventriculaire ne s'allonge par contre que modérément et les effets atteignent rapidement un plateau maximum dans la gamme de doses étudiée.

3. Tests antiarythmiques.

Les dérivés suivant l'invention sont utilisés dans le test à l'aconitine sur le rat. Pour ce test, le rat anesthésié est intoxiqué par une perfusion intraveineuse d'aconitine (solution de nitrate d'aconitine : 15 mg/l ; vitesse de perfusion : 0,4 ml/mn) tandis que son électrocardiogramme est enregistré en permanence. Durant la perfusion à vitesse constante, on mesure le temps nécessaire à l'apparition d'arythmies ventriculaires, successivement extrasystoles ventriculaires (ESV), tachycardie ventriculaire

(TV) et fibrillation ventriculaire (FV).

Les animaux sont répartis en lot témoin (non traité) et lots traités (différentes doses). Les résultats sont exprimés en pourcentage d'allongement du délai d'apparition des arythmies dans les lots traités par rapport au lot témoin. Cet allongement du délai d'apparition des arythmies correspond donc à une protection myocardique contre les effets arythmiques de l'aconitine.

Le tableau 4 ci-après regroupe les résultats obtenus avec divers dérivés conformes à la présente invention.

Tableau 4

| Exemple | Dose injectée mg/kg | ARYTHMIES VENTRICULAIRES (%) | | |
|---|---|---|---|---|
| | | ESV | TV | FV |
| 2 | 10 | + 32 | + 30 | + 44 |
| 3 | 10 | + 6 | + 28 | + 46 |
| 4 | 10 | + 7 | + 22 | + 18 |
| 6 | 3 | + 13 | + 21 | + 18 |
| 7 | 10 | + 11 | + 45 | + 32 |
| 9 | 10 | 0 | + 28 | + 38 |
| 11 | 10 | + 10 | + 11 | + 18 |
| 18 | 10 | + 2 | + 6 | 0 |
| 19 | 10 | + 15 | + 15 | + 35 |

L'activité antifibrillatoire cardiaque des dérivés conformes à l'invention a été vérifiée sur la souris au moyen du test de Lawson, suivant la méthode décrite par J. W. Lawson, J. Pharmacol. Exp. Therp. 1968, *160*, 22-31 et Cr. Narcisse et al., Ann. Pharm. Fr. 1979, *37*, 325-330.

Les souris (20 par dose) reçoivent une injection intrapéritonéale du dérivé 10 minutes avant d'être placées en atmosphère saturée en chloroforme. Dès l'arrêt respiratoire obtenu, le thorax est ouvert (5 à 10 s.) et on vérifie l'état du cœur : présence ou non d'une fibrillation ventriculaire. La dose efficace 50 (DE 50) du dérivé étudié est la dose qui protège la moitié des souris contre la fibrillation ventriculaire anoxique.

Les résultats du tableau 5 ci-après montrent que les dérivés suivant l'invention possèdent une bonne activité antifibrillatoire.

Tableau 5

| Dérivé | DE 50 (mg/kg) |
|---|---|
| 3 | 35 |
| 4 | 46 |
| 5 | 79 |
| 7 | 66 |
| 9 | 44 |
| 11 | 42,5 |
| 14 | 62 |
| 19 | 21,5 |

Par ailleurs, le test de Harris, effectué sur le chien suivant la technique décrite dans Circulation, 1950, *1*, 1318, montre que les dérivés suivant l'invention entraînent une diminution des extrasystoles ventriculaires. A titre d'exemple, le nombre d'extrasystoles ventriculaires par minute diminue de 50 % environ pendant les 2 h qui suivent l'injection du produit, dans le cas du dérivé décrit dans l'exemple 10. De plus l'effet antiarythmique (protection myocardique) augmente avec la dose injectée.

Enfin, on peut constater que les dérivés suivant la présente invention présentent un pouvoir anesthésique local vérifié sur la cornée de lapin (anesthésie locale de contact) après instillation d'un collyre contenant le dérivé à tester. Par exemple, les dérivés décrits aux exemples 2, 3, 4, 6, 7 et 9 sont anesthésiques locaux (anesthésie à 100 %) en solution à 0,25 % ou 1 %.

Ces résultats montrent que les dérivés du type aryl-3 alcoxy-5 pyrazole conformes à la présente invention possèdent des propriétés antiarythmiques intéressantes, ainsi que des propriétés électrophysiologiques et une bonne tolérance hémodynamique, permettant d'envisager leur application en thérapeutique humaine et vétérinaire, en particulier dans le traitement de diverses formes d'arythmies cardiaques.

Les dérivés conformes à l'invention et leurs sels pharmaceutiquement acceptables peuvent être administrés sous les formes usuelles, le principe actif étant dilué dans un support pharmaceutiquement acceptable convenablement choisi, par exemple sous forme de comprimé, gélule, dragée, suppositoire, soluté injectable au sirop.

A titre d'exemple, les comprimés peuvent être préparés en mélangeant le dérivé suivant l'invention ou un de ses sels, avec un ou plusieurs diluants solides tels que le lactose, le mannitol, l'amidon, la polyvinylpyrrolidone, le stéarate de magnésium, le talc, etc. Le cas échéant, les comprimés peuvent comporter plusieurs couches superposées autour d'un noyau, suivant les techniques usuelles, pour assurer une libération progressive ou un effet retardé du principe actif. L'enrobage peut par exemple être constitué d'une ou plusieurs couches d'acétate de polyvinyle, de carboxyméthylcellulose ou d'acétophtalate de cellulose.

On peut également administrer le dérivé suivant l'invention sous forme d'un sirop ou d'un soluté buvable obtenu en le dissolvant, le cas échéant sous forme de sel pharmaceutiquement acceptable, dans de l'eau ou du glycérol, par exemple, et en ajoutant au besoin un additif usuel tel qu'un édulcorant et un antioxydant.

Des solutions injectables peuvent être préparées suivant les techniques bien connues et sont constituées par exemple par un soluté contenant un dérivé suivant l'invention ou un de ses sels pharmaceutiquement acceptables, dissous dans de l'eau bidistillée, une solution hydroalcoolique, du propylèneglycol, etc., ou un mélange de ces solvants. Le cas échéant, un additif approprié tel qu'un conservateur peut être ajouté.

La posologie peut varier suivant la nature de l'affection traitée, et le sujet concerné. Les doses administrées journellement sont généralement comparables à celles des traitements quinidiniques, mais peuvent être ajustées par le praticien en fonction des circonstances.

**Revendications**

1. Aryl-3 alcoxy-5 pyrazoles, caractérisés en ce qu'ils sont représentés par la formule générale (I)

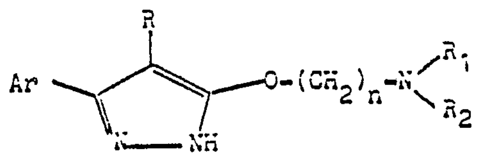

(I)

dans laquelle Ar représente un groupe phényle, ou un groupe phényle substitué par un ou plusieurs atomes d'halogène ou groupes alkyle, alcoxy, cyano, nitro, ou hydroxy ; R représente un atome d'hydrogène ou un groupe alkyle, phényle ou benzyle ; $R_1$ et $R_2$, identiques ou différents, représentent un atome d'hydrogène ou un groupe alkyle inférieur de 1 à 4 atomes de carbone, ou forment avec l'atome d'azote auquel ils sont rattachés, un hétérocycle de 5 à 7 chaînons comportant le cas échéant un ou plusieurs autres hétéroatomes choisis parmi l'azote et l'oxygène ; n est un entier de 1 à 4 ; ainsi que leurs sels d'acides.

2. Aryl-3 alcoxy-5 pyrazoles selon la revendication 1, caractérisés en ce que Ar est un groupe phényle, ou un groupe phényle substitué par un ou plusieurs atomes de chlore.

3. Aryl-3 alcoxy-5 pyrazoles selon la revendication 2, caractérisés en ce que Ar est un groupe phényle, un groupe p-chlorophényle ou un groupe 3,4-dichlorophényle.

4. Aryl-3 alcoxy-5 pyrazoles selon la revendication 1, caractérisés en ce que R est un atome d'hydrogène, un groupe alkyle inférieur de 1 à 4 atomes de carbone, ou un groupe benzyle.

5. Aryl-3 alcoxy-5 pyrazoles selon la revendication 1, caractérisés en ce que $R_1$ et $R_2$ forment avec l'atome d'azote auquel ils sont rattachés, un groupe pyrrolyle, pyrrolidinyle, pipéridyle, azépinyle, oxazolidinyle, pyrazolyle, pipérazinyle, imidazolyle, morpholinyle, ou pyrazolinyle.

6. Composition pharmaceutique caractérisée en ce qu'elle contient un aryl-3 alcoxy-5 pyrazole selon l'une quelconque des revendications précédentes, ou un de ses sels pharmaceutiquement acceptables, dilué le cas échéant dans un support approprié.

7. Procédé de préparation d'aryl-3 alcoxy-5 pyrazoles selon la revendication 1, caractérisé en ce qu'on fait réagir une pyrazolone de formule générale (II)

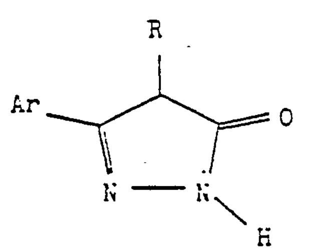

(II)

dans laquelle Ar et R ont la même signification que dans la formule (I), en milieu basique, avec une halogénoalkylamine de formule générale (III) :

$$X—(CH_2)_n—NR_1R_2 \qquad (III)$$

dans laquelle X est un atome d'halogène, et n, $R_1$ et $R_2$ conservent les définitions indiquées dans la formule (I).

8. Procédé selon la revendication 7, caractérisé en ce que la réaction s'effectue en présence d'une base choisie parmi un hydrure, un amidure, un carbonate ou un alcoolate de métal alcalin ou une amine, dans un solvant organique.

9. Procédé selon l'une quelconque des revendications 7 et 8, caractérisé en ce qu'on dissout la pyrazolone de formule (II) dans un solvant organique sous atmosphère d'azote, on ajoute une base, puis on ajoute l'halogénoalkylamine de formule (III).

10. Procédé de préparation d'aryl-3 alcoxy-5 pyrazoles selon la revendication 1, caractérisé en ce qu'on fait réagir un aroylacétate d'alkyle de formule générale (IV) :

$$Ar-\underset{\underset{O}{\|}}{C}-CHR-\underset{\underset{O}{\|}}{C}-OC_2H_5 \qquad (IV)$$

où Ar et R ont la même signification que dans la formule (I), avec un aminoalcool de formule générale (V) :

$$HO—(CH_2)_n—NR_1R_2 \qquad (V)$$

où n, $R_1$ et $R_2$ ont la même signification que dans la formule (I), pour obtenir un composé de formule (VI) :

$$Ar-\underset{\underset{O}{\|}}{C}-CHR-\underset{\underset{O}{\|}}{C}-(CH_2)_n-NR_1R_2 \qquad (VI)$$

sur lequel on effectue une cyclisation au moyen d'hydrazine en milieu acide.

11. Procédé selon la revendication 10, caractérisé en ce que la réaction de l'aroylacétate d'éthyle de formule (IV) et de l'aminoalcool de formule (V), s'effectue dans un solvant permettant une distillation azéotropique.

**Claims**

1. 3-aryl-5-alkoxy-pyrazoles, characterized in that they are represented by general formula (I)

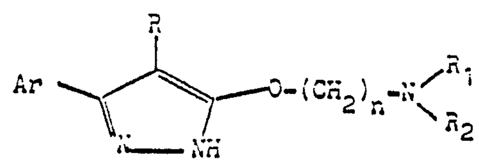

(I)

wherein Ar represents a phenyl group, or a phenyl group substituted with one or more halogen atoms or alkyl, alkoxy, cyano, nitro, or hydroxy group ; R represents a hydrogen atom or an alkyl, phenyl or benzyl group ; $R_1$ and $R_2$, which may be the same or different, each represent a hydrogen atom or a lower alkyl group with 1 to 4 carbon atoms, or together form, with the nitrogen atom to which they are attached, a 5 to 7 membered heterocyclic ring, which possibly comprises one or more heteroatoms selected from nitrogen and oxygen ; n is an integer of from 1 to 4 ; and the acid salts thereof.

2. 3-aryl-5-alkoxy-pyrazoles of claim 1, characterized in that Ar is a phenyl group or a phenyl group substituted with one or more chlorine atoms.

3. 3-aryl-5-alkoxy pyrazoles of claim 2, characterized in that Ar is a phenyl group, a p-chlorophenyl group or a 3,4-dichlorophenyl group.

4. 3-aryl-5-alkoxy-pyrazoles of claim 1, characterized in that Ar is a hydrogen atom, a lower alkyl group with 1 to 4 carbon atoms, or a benzyl group.

5. 3-aryl-5-alkoxy-pyrazole of claim 1, characterized in that $R_1$ and $R_2$ form, with the nitrogen atom to which they are attached, a pyrrolyl, pyrrolidinyl, piperidyl, azepinyl, oxazolidinyl, pyrazolyl, piperazinyl, imidazolyl, morpholinyl or pyrazolinyl group.

6. A pharmaceutical composition characterized in that it contains a 3-aryl-5-alkoxy-pyrazole according to any of the preceding claims, or a pharmaceutically acceptable salt thereof, possibly diluted in an acceptable carrier.

7. A process for the preparation of 3-aryl-5-alkoxy-pyrazoles of claim 1, characterized in that it comprises reacting a pyrazolone of general formula (II) :

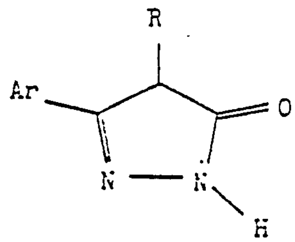

(II)

wherein Ar and R have the same meaning as in formula (I), in a basic medium with a haloalkylamine of general formula (III) :

$$X—(CH_2)_n—NR_1R_2 \qquad (III)$$

wherein X is a halogen atom, and n, $R_1$ and $R_2$ have the definitions given in formula (I).

8. The process of claim 7, characterized in that the reaction is carried out in the presence of a basic medium selected from a hydride, an amide, a carbonate, an alkali metal alkoxide or an amine, in an organic solvent.

9. The process according to any of claims 7 and 8, characterized in that the pyrazolone of formula (II) is dissolved in an organic solvent under a nitrogen atmosphere, a base is added, and then the haloalkylamine of formula (III) is added.

10. A process for the preparation of 3-aryl-5-alkoxy-pyrazoles of claim 1, characterized in that it comprises reacting an alkyl aroylacetate of general formula (IV) :

$$Ar-\underset{\underset{O}{\|}}{C}-CHR-\underset{\underset{O}{\|}}{C}-OC_2H_5 \qquad (IV)$$

wherein Ar and R have the same meaning as in formula (I) with an aminoalcohol of general formula (V) :

$$HO—(CH_2)_n—NR_1R_2 \qquad (V)$$

wherein n, $R_1$ and $R_2$ have the same meaning as in formula (I), to obtain a compound of formula (VI) :

$$Ar-\underset{\underset{O}{\|}}{C}-CHR-\underset{\underset{O}{\|}}{C}-(CH_2)_n-NR_1R_2 \qquad (VI)$$

and cyclizing the compound of the formula (VI) using hydrazine in an acid medium.

11. The process of claim 10, characterized in that the reaction of the ethyl aroylacetate of formula (IV) and the aminoalcohol of formula (V) is carried out in a solvent enabling azeotropic distillation.

**Patentansprüche**

1. 3-Aryl-5-alkoxy-pyrazole, gekennzeichnet durch die allgemeine Formel (I)

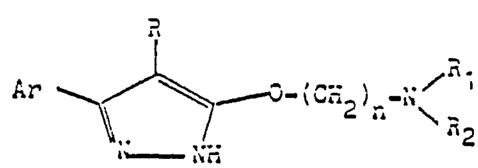

(I)

worin bedeuten : Ar eine Phenylgruppe oder eine durch ein(e) oder mehrere Halogenatome, Alkyl-, Alkoxy-, Cyano-, Nitro- oder Hydroxygruppen substituierte Phenylgruppe ; R ein Wasserstoffatom oder eine Alkyl-, Phenyl- oder Benzylgruppe ; $R_1$ und $R_2$, die gleich oder verschieden sein können, ein Wasserstoffatom oder eine Niederalkylgruppe mit 1 bis 4 Kohlenstoffatomen, oder die mit dem Stickstoffatom, an dem sie gebunden sind, einen 5- bis 7-gliedrigen Heterozyklus bilden können, der gegebenenfalls ein oder mehrere andere Heteroatome, gewählt aus Stickstoff und Sauerstoff, enthalten kann ; n eine ganze Zahl von 1 bis 4 ; sowie deren Säuresalze.

2. 3-Aryl-5-alkoxy-pyrazole nach Anspruch 1, dadurch gekennzeichnet, daß Ar eine Phenylgruppe oder eine durch ein oder mehrere Chloratome substituierte Phenylgruppe bedeutet.

3. 3-Aryl-5-alkoxy-pyrazole nach Anspruch 2, dadurch gekennzeichnet, daß Ar eine Phenylgruppe, eine p-Chlorphenylgruppe oder eine 3,4-Dichlorphenylgruppe bedeutet.

4. 3-Aryl-5-alkoxy-pyrazole nach Anspruch 1, dadurch gekennzeichnet, daß R ein Wasserstoffatom, eine Niederalkylgruppe mit 1-4 Kohlenstoffatomen oder eine Benzylgruppe bedeutet.

5. 3-Aryl-5-alkoxy-pyrazole nach Anspruch 1, dadurch gekennzeichnet, daß $R_1$ und $R_2$ mit dem Stickstoffatom, an dem sie gebunden sind, eine Pyrrolyl-, Pyrrolidinyl-, Piperidyl-, Azepinyl-, Oxazolidinyl-, Pyrazolyl-, Piperazinyl-, Imidazolyl-, Morpholinyl- oder Pyrazolinylgruppe bilden.

6. Pharmazeutische Zusammensetzung, dadurch gekennzeichnet, daß sie ein 3-Aryl-5-alkoxy-pyrazol nach einem der vorangehenden Ansprüche oder eines dessen pharmazeutisch annehmbarer Salze, gegebenenfalls in Verdünnung mit einem geeigneten Träger, enthält.

7. Verfahren zur Herstellung der 3-Aryl-5-alkoxy-pyrazole nach Anspruch 1, dadurch gekennzeichnet, daß man ein Pyrazolon der allgemeinen Formel (II)

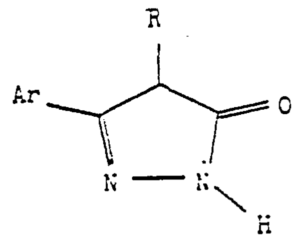

(II)

worin Ar und R die gleiche Bedeutung wie in der Formel (I) besitzen, in basischem Milieu mit einem Halogenalkylamin der allgemeinen Formel (III)

$$X-(CH_2)_n-NR_1R_2 \qquad \text{(III)}$$

worin X ein Halogenatom darstellt und n, $R_1$ und $R_2$ den bei der Formel (I) angegebenen Definitionen entsprechen, umsetzt.

8. Verfahren nach Anspruch 7, dadurch gekennzeichnet, daß die Umsetzung in Gegenwart einer Base, gewählt aus einem Hydrid, Amid, Carbonat oder Alkoholat eines Alkalimetalls oder einem Amin, in einem organischen Lösungsmittel durchgeführt wird.

9. Verfahren nach Anspruch 7 und/oder 8, dadurch gekennzeichnet, daß man das Pyrazolon der Formel (II) in einem organischen Lösungsmittel unter Stickstoffatmosphäre löst, eine Base und daran anschließend das Halogenalkylamin der Formel (III) zugibt.

10. Verfahren zur Herstellung der 3-Aryl-5-alkoxy-pyrazole nach Anspruch 1, dadurch gekennzeichnet, daß man ein Alkylaroylacetat der allgemeinen Formel (IV)

$$Ar-\underset{O}{\underset{\|}{C}}-CHR-\underset{O}{\underset{\|}{C}}-OC_2H_5 \qquad \text{(IV)}$$

worin Ar und R die gleiche Bedeutung wie in der Formel (I) besitzen, mit einem Aminoalkohol der allgemeinen Formel (V)

$$HO-(CH_2)_n-NR_1R_2 \qquad \text{(V)}$$

worin n, $R_1$ und $R_2$ die gleiche Bedeutung wie in Formel (I) besitzen, umsetzt, um eine Verbindung der Formel (VI)

$$Ar-\underset{O}{\underset{\|}{C}}-CHR-\underset{O}{\underset{\|}{C}}-(CH_2)_n-NR_1R_2 \qquad \text{(VI)}$$

zu erhalten, bei der man mittels Hydrazin im sauren Milieu eine Zyklisierung durchführt.

11. Verfahren nach Anspruch 10, dadurch gekennzeichnet, daß man die Umsetzung des Ethylaroylacetats der Formel (IV) mit dem Aminoalkohol der Formel (V) in einem Lösungsmittel durchführt, das eine azeotrope Destillation gestattet.